(19)

Europäisches
Patentamt
European
Patent Office
Office européen
des brevets

(11) **EP 4 105 941 A1**

(12) **EUROPEAN PATENT APPLICATION**

(43) Date of publication:
**21.12.2022 Bulletin 2022/51**

(21) Application number: **21179681.8**

(22) Date of filing: **16.06.2021**

(51) International Patent Classification (IPC):
**G16H 50/20** (2018.01)        **G16H 50/30** (2018.01)

(52) Cooperative Patent Classification (CPC):
**G16H 50/30; G16H 50/20;** G16H 20/30;
G16H 20/60; G16H 20/70

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicants:
• **Kauno Technologijos Universitetas**
  **44029 Kaunas (LT)**
• **Vilniaus Universitetas**
  **01513 Vilnius (LT)**

(72) Inventors:
• **Petrenas, Andrius**
  **50380 Kaunas (LT)**
• **Butkuviene, Monika**
  **47222 Kaunas (LT)**
• **Paliakaite, Birut**
  **44313 Kaunas (LT)**

• **Bacevicius, Justinas**
  **09311 Vilnius (LT)**
• **Plusciauskaite, Vilma**
  **51381 Kaunas (LT)**
• **Solosenko, Andrius**
  **51352 Kaunas (LT)**
• **Daukantas, Saulius**
  **48146 Kaunas (LT)**
• **Sokas, Daivaras**
  **50449 Kaunas (LT)**
• **Rapalis, Andrius**
  **51290 Kaunas (LT)**
• **Aidietis, Audrius**
  **14263 Vilnius distrc. (LT)**
• **Marozas, Vaidotas**
  **51361 Kaunas (LT)**

(74) Representative: **Pakeniene, Ausra**
  **AAA Law**
  **A. Gostauto street 40B**
  **03163 Vilnius (LT)**

(54) **METHOD FOR ESTABLISHING A CAUSALITY SCORE BETWEEN ATRIAL FIBRILLATION TRIGGERS AND ATRIAL FIBRILLATION PATTERN**

(57)    Method for establishing a causality score between atrial fibrillation triggers and atrial fibrillation occurrence pattern for use for management of personally identified atrial fibrillation triggers in relation to temporal atrial fibrillation occurrence patterns, the method steps are realized in the software modules distributed between personal computing device and cloud-based server wherein the method steps comprise detecting (101) time-specific AF trigger occurrence instance in biosignals, enrolling (102) data representing reported AF triggers, transforming (103) the enrolled data representing AF triggers to time-series signals, constructing (104) temporal AF episode occurrence patterns, characterizing (105) temporal AF episode occurrence patterns with respect to episode aggregation or/and clustering, matching (106) AF trigger and temporal AF episode occurrence pattern data, calculating (107) the causality score between the suspected AF triggers and the temporal AF episode occurrence pattern for identifying personal AF triggers.

Fig. 1

Processed by Luminess, 75001 PARIS (FR)

## Description

Technical field

The present invention relates to methods for assessment of a person's health risks based on the person's biosignals, and more particularly, to a method for establishing a causality score between person's atrial fibrillation triggers and atrial fibrillation pattern. The causality score might be useful in nonpharmacological self-management of the disease in its early stages by inspiring lifestyle behaviour changes.

Background art

Despite advances in arrhythmia treatment technology, atrial fibrillation (further - AF) remains a worldwide cardiovascular epidemic affecting more than 33 million individuals globally. AF carries a 5-fold increased risk of brain stroke, 3-fold increased risk of heart failure and causes up to 40% of annual hospitalizations of AF patients. For these reasons, AF imposes a substantial economic burden, e.g., the total healthcare costs of AF reach 2.6% of healthcare expenditure in Europe. Given that AF is especially common among >65-year-olds, the prevalence is expected to increase up to 3-fold in the upcoming decades due to the ageing society. AF is a progressive disease with primary self-terminating (paroxysmal) episodes often lasting from less than 30 s to 7 days. Therefore, treatment success and complication management highly depend on what stage of arrhythmia development AF is detected. Paroxysmal AF may terminate by itself, whereas persistent AF, lasting longer than seven days, may be terminated via pharmacological or electrical cardioversion. Considering that prolonged AF causes electrical and structural remodelling of the atria, it is desirable to maintain arrhythmia-free heart rhythm for as long as possible. Acute exposures, hereinafter AF triggers, such as unhealthy habits, specific physical activities, foods, medications, etc., may contribute to the initiation of AF episodes either in the absence or presence of already existing electrical or structural atrial remodelling. Evidence supports the links between AF occurrence and alcohol, caffeine, physical exertion, sleep disorders. For instance, Groh et al., "Patient-reported triggers of paroxysmal atrial fibrillation", Heart Rhythm, 16.7, July 2019, pages 996-1002, disclosed a list of patient-reported AF triggers, identified by asking study participants to complete a questionnaire regarding their cardiovascular risk factors as well as suspected AF triggers. Similarly, by gathering information using questionnaires, Hansson et al., "Arrhythmia-provoking factors and symptoms at the onset of paroxysmal atrial fibrillation", BMC Cardiovascular Disorders, 4.1, December 2004, pages 13, disclosed self-reported symptoms and AF triggering factors. Based on the patient reported lists, many of AF triggers are modifiable, suggesting behavioural interventions, e.g., avoidance of particular triggers, as potentially effective in maintaining arrhythmia-free heart rhythm.

Temporal patterns of self-terminating AF episodes may be established by collecting biosignals of a person using either implantable devices or external electrocardiogram (ECG) recorders with dedicated software to detect AF. Implantable devices, such as implantable loop recorders, pacemakers, cardioverters-defibrillators, biventricular pacemakers for cardiac resynchronization therapy, may be programmed to detect AF by analysing intra-atrial electrogram acquired directly from the heart via an atrial lead. However, implantable devices are costly and risky due to the implantation procedure. Meanwhile, non-invasive devices for long-term AF monitoring, such as conventional Holter monitors and modern ECG patches (e.g., Zio XT Patch or ZIO Event Card, iRhythm Tech., Inc., San Francisco, CA; Bittium Faros, Bittium, Inc., Oulu, Finland), are inconvenient due to skin irritation. Alternatively, irregular heart rhythm, which may indicate AF, may be detected in optical photoplethysmogram (PPG) signals, acquired by most smartwatches. A few smartwatches, such as Apple Watch, Huawei Watch GT2, Samsung Galaxy Active 2, and Samsung Galaxy Watch 3, offer the possibility to detect AF. However, the aforementioned smartwatches do not look for AF continuously, thus are unsuitable for establishing temporal AF occurrence patterns. As a solution to this limitation, Solosenko et al., "Detection of atrial fibrillation using a wrist-worn device", Physiological Measurement, 40.2, February 2019, pages 025003, proposed a low-complexity algorithm for long-term AF monitoring, designed to analyse PPG signals, acquired using wrist-worn devices.

AF treatment (e.g., antiarrhythmic drugs, cardioversion, cardiac ablation, oral anticoagulants to prevent blood clot formation) is commonly prescribed relying on a binary outcome, i.e., the presence or absence of AF in the ECG. However, awareness is growing that the risk of complications may be better predicted relying on quantitative measures that account to the properties of temporal AF episode occurrence pattern. For instance, 2020 ESC Guidelines for the diagnosis and management of AF, European Heart Journal, 42.5, February 2021, pages 373-498, discuss the structured stratification of AF patients using 4S-AF scheme which includes stroke risk, symptom severity, atrial substrate severity, and severity of AF episode burden. Given that AF burden, which is the percentage of time spent in AF during the monitored period, do not account for episode clustering, another way to quantify AF occurrence pattern is to find the temporal distribution of self-terminating AF episodes, e.g., by finding the aggregation of AF burden. For instance, Charitos et al., "Atrial fibrillation density: A novel measure of atrial fibrillation temporal aggregation for the characterization of atrial fibrillation recurrence pattern", Applied Cardiopulmonary Pathophysiology, 17, January 2013, pages 3-10, disclosed a measure "AF density" to describe the temporal AF occurrence pattern where low AF density values reflect low temporal AF burden aggregation, whereas high values reflect high temporal AF burden aggregation. Yet another way to quantify AF occurrence pattern is to assess the degree of AF episode clustering. For instance, Henriksson et al., "Modeling and estimation of temporal episode patterns in paroxysmal atrial fibrillation", IEEE Transactions on Biomedical Engineering, 68.1, January 2021, pages 319-329, disclosed a model-based, statistical approach to characterize episode patterns of paroxysmal AF where model parameters depend on AF episode clustering. This approach is highly sensitive to the duration of the monitoring period and requires a certain number of AF episodes, e.g., at least 10, to provide reliable estimates.

The available approaches to AF treatment and complication management are associated with serious side effects and costs. However, scientific evidence shows that AF burden and AF recurrence may be decreased by lifestyle and risk factor modification. For instance, Voskoboinik et al., "Alcohol abstinence in drinkers with atrial fibrillation", New England Journal of Medicine, 382.1, January 2020, pages 20-28, reported the reduction of AF recurrence and decrease in AF burden by avoiding alcohol. Accordingly, Chung et al., "Lifestyle and risk factor modification for reduction of Atrial Fibrillation", Circulation, 141.16, April 2020, pages e750-e772, discussed a framework for a structured, protocol-driven approach to include management of risk factors as an integral part of AF treatment.

Establishing a causal relationship between AF triggers and temporal AF episode occurrence pattern for use for a person's individual-focused control of AF triggers may be an effective strategy to achieve nonpharmacological AF management or to supplement conventional pharmaceutical treatment.

United States patent No. US 9420956 B2 and the following United States patent application US 2020/0229713 A1 disclose devices, methods and systems for managing cardiac diseases, including arrhythmias such as AF. The software application provided on a smartphone or tablet, includes a cardiac health score that can be calculated in response to user's data such as ECG, personal information and risk factors. The system can log behaviours and activities, such as consuming foods, drinks, medications, which are associated with abnormal ECG recordings and inform the patient about these associations. The system can also provide recommendations to avoid these behaviours and activities or set daily goals to meet and thereby improve cardiac health score. The goals may be determined by the user or a medical staff member and updated as cardiac health score changes.

United States patent application US 2019/0008384 A1 discloses systems and methods for managing device-generated medical events detected from patients. A medical event management system includes an event analyser circuit to detect a medical event using physiological data from a patient-triggered episode acquired from a medical device. Physiological data may include ECG, heart rate, atrial and ventricular parameters, blood oxygen saturation, physical activity, physiological response to activity, posture, respiratory weight, body temperature, etc. The system may include patient-reported sign or symptom next to the patient-triggered episode. Based on the temporal relationship between the patient-reported sign or symptom and the detected medical event, the confidence score is determined.

United States patent US 10726545 B2 discloses methods and systems for assessing the risk of thromboembolic stroke in AF patients. The system comprises a processing module that determines a degree of fibrosis of a patient's left atrium relying on the image data. As a result of image analysis, treatment is determined and administered to the patient.

International patent application WO 2020/104986 A2 discloses a system and a method intended to noninvasively detect, monitor and characterize atrial arrhythmias. The system consists of a portable device with integrated biosensors, the modules integrated in the portable device to detect intermittent atrial arrhythmia episodes, and a module to characterize the distribution of atrial arrhythmia episodes. The biooptical sensor is integrated into the wearable device to acquire PPG signal continuously. The integrated biopotential electrodes acquire a short-term single lead ECG or ECGs of the modified I, II, III, aVR, aVL, aVF leads on demand. The system is designed for use in the server or smart device. If a non-documented atrial arrhythmia, especially AF, is detected during the long-term monitoring, a medical staff member is informed by an email by sending the ECG of the arrhythmic episode.

European patent application EP 3654347 A1 discloses a system and a method for detecting and monitoring life-threatening conditions due to electrolyte fluctuations in blood serum. The system comprises a wrist-worn device with integrated biosignal sensors, real-time signal processing modules integrated in the worn device, and a sudden death risk-assessment module that is used in a server, a personal computer, or a smart device. Life-threatening arrhythmias are detected by analysing a continuously acquired PPG signal, whereas electrolyte fluctuations are monitored by analysing a short-term ECG signal acquired by integrated biopotential sensors. The system allows to collect other information related to a medical condition by means of a smart device. Detailed information about the patient's health status and the risk of life-threatening conditions may be sent to a medical staff member.

Brief description of the invention

The present invention provides a method for establishing a causality score between person's AF triggers (e.g., alcohol, caffeine, physical exertion) and a person's temporal AF episode occurrence pattern which may comprise of a single self-terminating AF episode, several episodes, a cluster, or several clusters of AF episodes.

The method may be implemented as a distributed set of software modules on a personal computing device such as a standard personal computer, a portable computer, a handheld device, a smartphone, and cloud-based server. The personal computing device is responsible for collecting and pre-processing of the data, transmitting the data to the cloud server and presenting the results of analysis. The cloud-based server stores, synchronises, and analyses the data collected. Finally, it sends the results of analysis back to the personal computing device.

The method comprises producing temporal AF episode occurrence patterns from continuously monitored biosignals, detecting AF triggers in biosignals, enrolling data representing person-reported AF triggers, transforming the data representing reported AF triggers to time-series signals, matching the time-series signals with the temporal AF episode occurrence pattern, calculating a causality score between the suspected AF triggers and the temporal AF episode occurrence pattern.

A module configured to detect AF triggers in biosignals analyses biosignals such as PPG, ECG, acceleration, etc. Biosignals might have been collected using various means such as wearable devices, such as a smartwatch, a bracelet, a chest-strap, and the like.

A module configured to collect information about person's reported AF triggers may be implemented as a software application in the personal computing device such as a smartwatch, a smartphone, a tablet, a laptop, a personal computer, or a further computing device such as a smartwatch, a smartphone, a tablet, a laptop, a personal computer. The reported AF triggers are entered by the person via a user interface comprising means for data input, representation, and data output. The reported AF triggers are time-stamped so that the reported AF triggers could be matched with the temporal AF episode occurrence pattern. The module may be configured to use further information extracted from biosignals of the biosensors or medical devices.

The user interface for registering reported AF triggers by the person is configured for entering suspected triggers and for sending feedback messages for the person. The feedback messages can be related to the characteristics of AF. A second user interface may be configured for a medical staff member for analysing person's information. The information may include person's data on time-stamped AF triggers and the AF episode occurrence pattern, AF pattern-characterizing parameters, person-reported and biosignal-detected AF triggers with the causality score calculated between each suspected trigger and the AF episode occurrence pattern.

To establish the causality score, person-reported AF triggers must be transformed from the time-stamped data to time-series signals. The unit-amplitude pulses, starting at the time instances the triggers are time-stamped by the person, are characterized by the growth functions with the growth rate parameters and by the decay functions with the decay rate parameters.

A module configured to collect temporal AF episode occurrence patterns may consist of a dedicated AF detection algorithm, capable of automatically analysing biosignals, such as PPG or ECG, with respect to AF-related signal properties.

AF episodes may manifest in clusters or aggregate within a specific time interval (e.g., during the night), therefore, AF episode patterns may be translated to pattern-characterizing parameters, e.g., that account to AF episode clustering or aggregation over time, to provide additional information on the influence of a specific trigger on AF development.

A software module configured to match the temporal AF episode occurrence pattern, or some pattern-characterizing parameter, with the suspected AF triggers includes data from the entire monitoring period. In case of person-reported AF triggers, the signal comprises specific time intervals with the transformed suspected AF triggers and outside-time intervals filled with zeros or with determined baseline values. In case of detected triggers, the signal consists of accumulated value within some time interval, e.g., hour, day, week.

A software module configured to establish the causal relationship between particular AF triggers and the temporal AF episode occurrence pattern generates the causality score using causality estimation methods, e.g., Granger causality. The causality score may be used to estimate the likelihood that the causal relationship between a particular trigger and the AF episode occurrence pattern exists. The identified causal relationship may be used for notification of the person and the medical staff member about the identified AF trigger which may have caused AF via software application implemented in some computing device (e.g., smartphone).

Brief description of the drawings

An exemplary approach for implementing nonpharmacological AF management through identification of AF triggers is illustrated by the accompanying drawings. Such implementation is demonstrative and neither intended to be exhaustive nor comprehensive.

FIG. 1 shows a block diagram of method steps according to the embodiment of the invention.
FIG. 2 shows an example of representation of relation of biosignals to particular AF triggers.

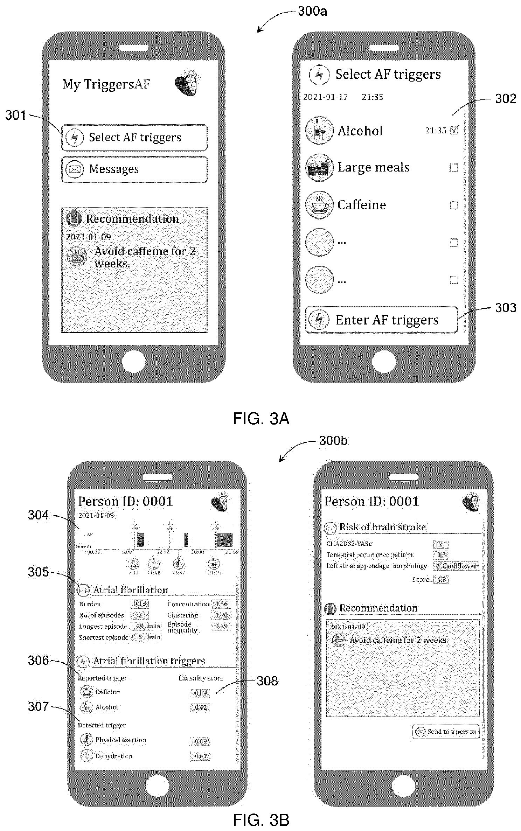

FIG. 3A shows an example of a user interface for enrolling data of person-reported AF triggers and receiving feedback concerning the enrolled AF triggers on a personal computing device.

FIG. 3B shows an example of a user interface on a further computing device for output of information on temporal AF episode occurrence patterns, detected AF triggers, the calculated causality score between the detected triggers and the temporal AF episode occurrence pattern.

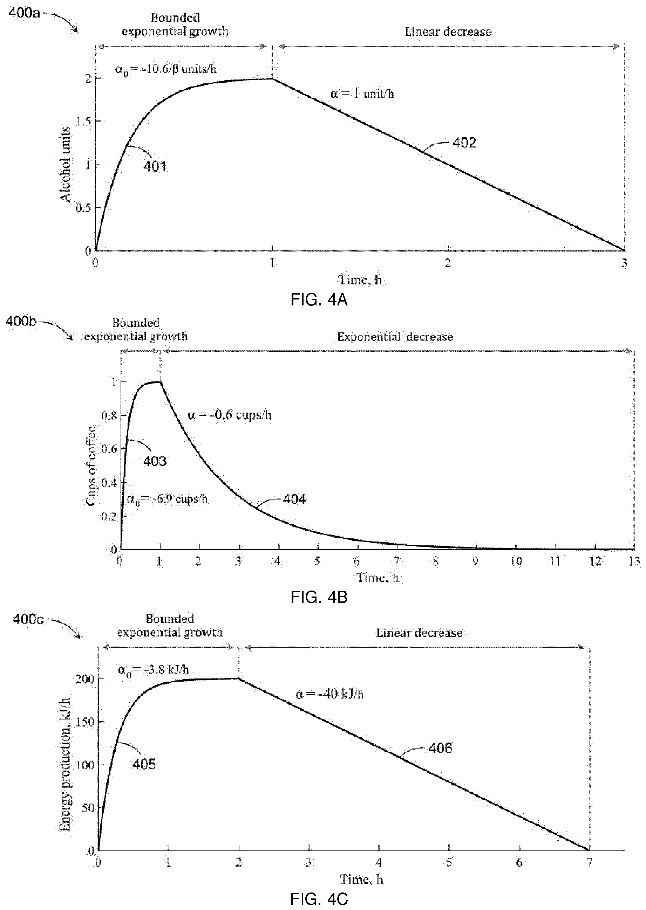

FIG. 4A shows the transformation of the person-reported AF trigger, in particular - alcohol, to the time-series signal.

FIG. 4B shows the transformation of the person-reported AF trigger, in particular - caffeine, to the time-series signal.

FIG. 4C shows the transformation of the person-reported AF trigger, in particular - large meal, to the time-series signal.

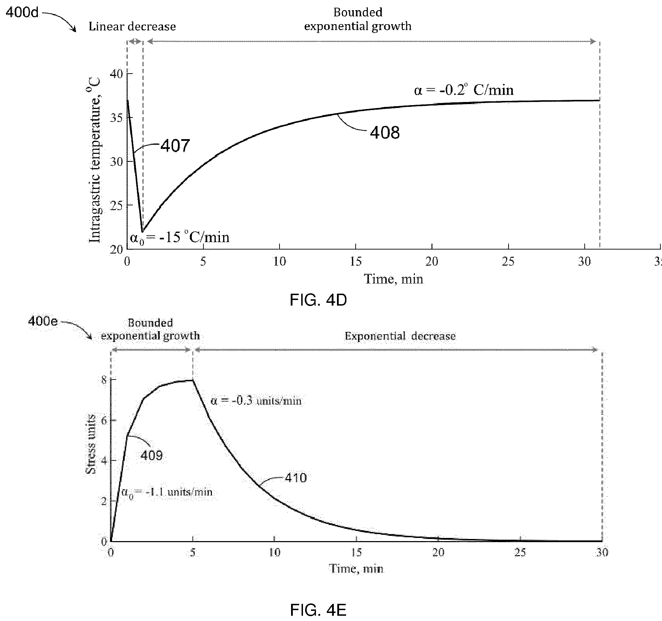

FIG. 4D shows the transformation of the person-reported AF trigger, in particular - cold beverage and food, to the time-series signal.

FIG. 4E shows the transformation of the person-reported AF trigger, in particular - acute emotional stress, to the time-series signal.

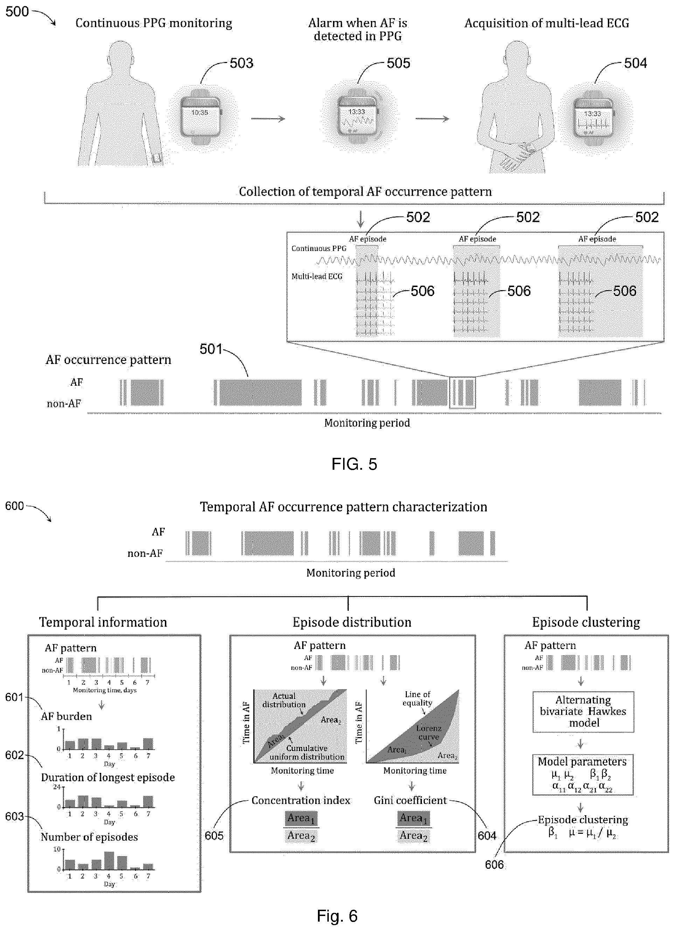

FIG. 5 shows an example of a module for collecting data on temporal AF episode occurrence patterns using a wrist-worn device capable of detecting AF episodes in long-term PPG signals.

FIG. 6 shows an example of a module for characterizing temporal AF episode occurrence patterns with respect to temporal AF episode information, episode distribution and episode clustering.

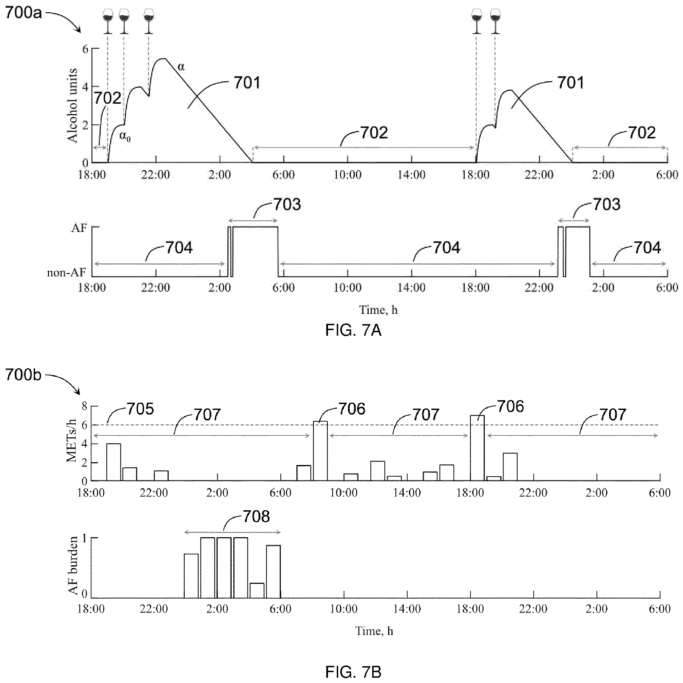

FIG. 7A shows an example of representation of alcohol consumption, transformed to the time-series signal, and the temporal AF episode occurrence pattern.

FIG. 7B shows an example of representation of physical activity expressed as METs per hour and the temporal AF episode occurrence pattern.

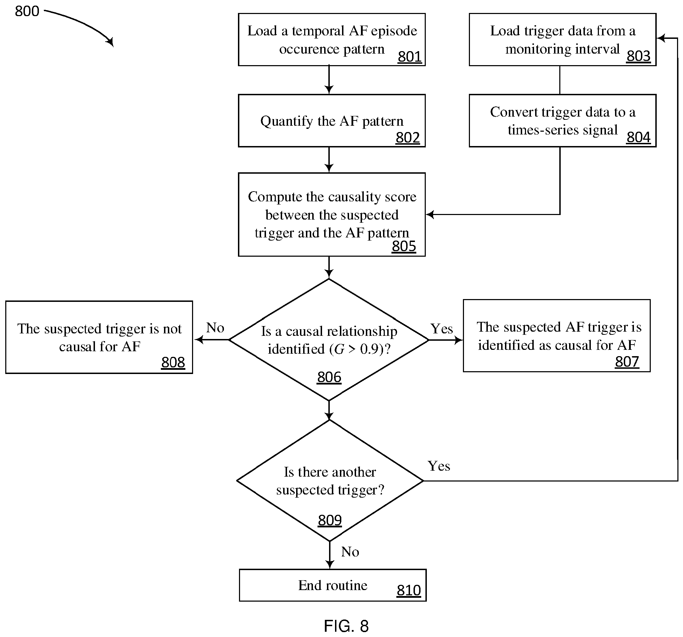

FIG. 8 shows an algorithm for determining the causality score between the suspected triggers and the temporal AF episode occurrence pattern.

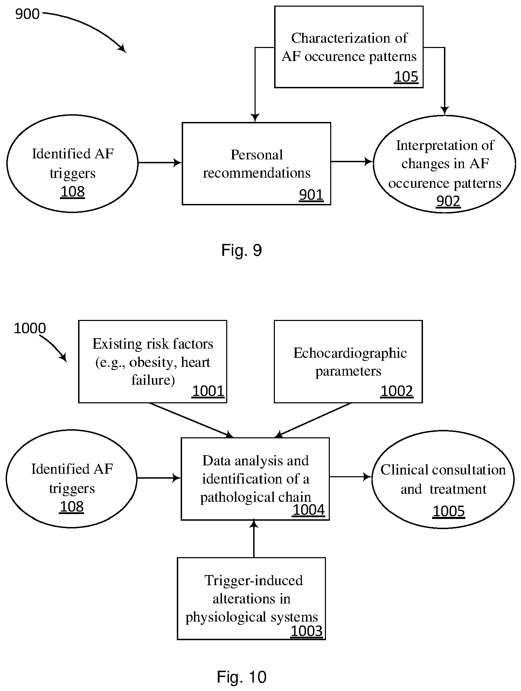

FIG. 9 shows a flow diagram illustrating an example implementation of the embodiment of the invention for modification of the temporal AF episode occurrence pattern by recommending avoiding identified AF triggers.

FIG. 10 shows a flow diagram illustrating a further example implementation of the embodiment of the invention for identification of a pathological chain of events leading to the occurrence of AF episodes.

FIG. 11A shows a flow diagram illustrating a further example implementation of the embodiment of the invention for identification of a pathological chain of events leading to AF occurrence in patients with obesity.

FIG. 11B shows a flow diagram illustrating a further example implementation of the embodiment of the invention for identification of a pathological chain of events leading to AF occurrence when mechanism of activated sympathetic component of the autonomic nervous system (ANS) is involved.

FIG. 11C shows a flow diagram illustrating a further example implementation of the embodiment of the invention for identification of a pathological chain of events leading to AF occurrence when mechanism of activated parasympathetic component of the ANS is involved.

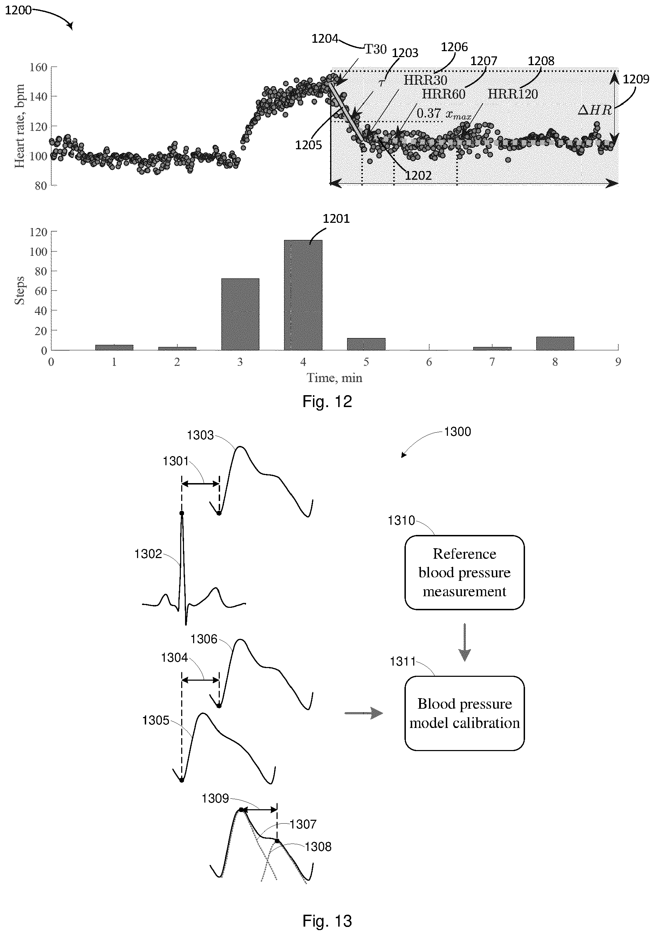

FIG. 12 shows a further example implementation of the embodiment of the invention for characterization of the ANS in free-living activities using wearable devices.

FIG. 13 shows an example of detection of changes in blood pressure using wearable devices.

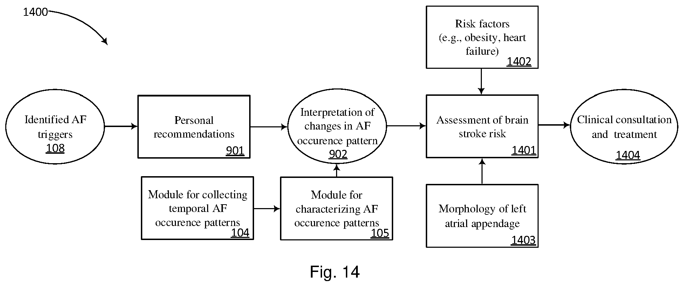

FIG. 14 shows a flow diagram illustrating a further example implementation of the embodiment of the invention for personal assessment of ischemic brain stroke risk based on existing risk factors, characteristics of the temporal AF episode occurrence pattern, and the morphology of the left atrial appendage.

Detailed description of the invention

Disclosed herein is a method (100) for establishing a causality score between suspected AF triggers and temporal AF episode occurrence pattern after paroxysmal or persistent AF is clinically diagnosed for a person. The method does not involve any method steps attributed to diagnostic methods.

The following terms are used to describe the embodiment of the invention:

- The term "AF trigger" denotes acute exposure that contributes to the occurrence of an AF episode or a cluster of episodes. AF trigger should not be understood as an AF symptom or risk factor.
- The term "reported AF trigger" denotes a suspected AF trigger, subjectively identified by a person as initiating an AF episode or a cluster of episodes, which is entered by a person and cannot be detected in biosignals. Examples of reported AF triggers are alcohol, caffeine, large meals, and the like.
- The term "detected AF trigger" denotes a suspected AF trigger, which may initiate AF episode or a cluster of episodes, and which is automatically detected in biosignals. Examples of detected triggers are physical exertion, sleep disorders, and the like.
- The term "temporal AF episode occurrence pattern" denotes a distribution of self-terminating AF episodes, occurring and terminating spontaneously within a specified time interval. Temporal AF occurrence pattern may comprise a single AF episode, several evenly spread episodes over a time period, a cluster of AF episodes, or several clusters of AF episodes spread over a time period.

- The term "identified trigger" denotes a suspected AF trigger which presence is causally related to the temporal AF episode occurrence pattern.

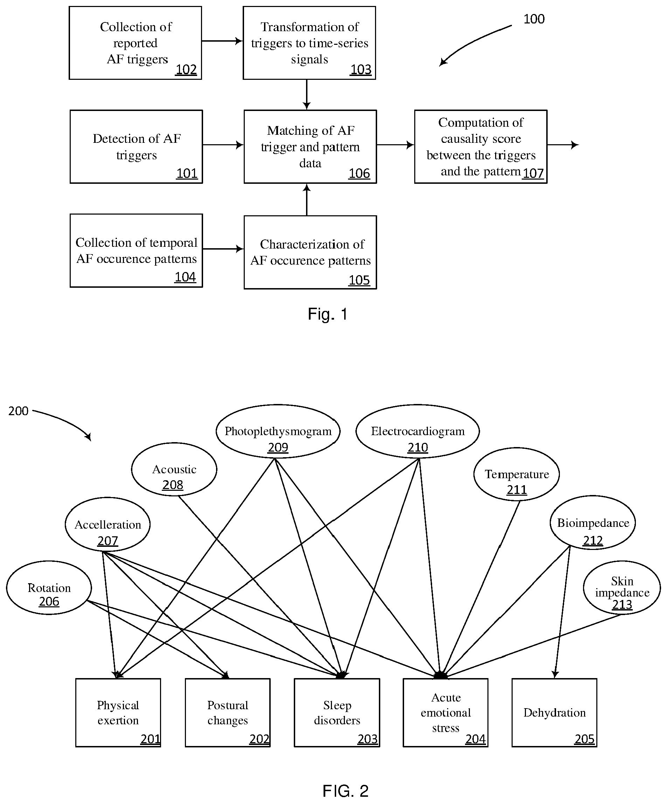

According to the embodiment of the invention and FIG. 1, steps of the method (100) are at least partially carried out on a personal computing device such as a standard personal computer, a portable computer, a handheld device, such as a smartphone, and the like. The method (100) steps comprises detecting (101) AF triggers in time-series biosignals, enrolling (102) data representing reported AF triggers, subjectively identified as initiating AF and entered by the person, transforming (103) the enrolled data representing AF triggers to time-series signals, constructing (104) temporal AF episode occurrence patterns, characterizing (105) temporal AF episode occurrence patterns with respect to episode aggregation or/and clustering, matching (106) AF trigger and temporal AF episode occurrence pattern data, calculating (107) the causality score between the suspected AF triggers and the temporal AF episode occurrence pattern. The causality score can be used for the identification of AF triggers, which presence is causally related to the temporal AF episode occurrence pattern.

A module configured to detect AF triggers (101) analyses biosignals acquired using one or more sensors. AF triggers may be detected in one or more biosignals such as rotation (206), acceleration (207), acoustic (208), PPG (209), ECG (210), temperature (211), body bioimpedance (212), skin impedance (213), etc. The module comprising of sensors for signal acquisition and software for signal processing, may be realized as a wrist-worn device (e.g., a smartwatch, a bracelet), a chest-strap, and the like. Examples of AF triggers to detect automatically in biosignals may include, but are not limited to, physical exertion (201), posture changes (202), sleep disorders (e.g., poor sleep quality, snoring, obstructive sleep apnea) (203), acute emotional stress (204), dehydration (205).

Examples of AF triggers detectable in biosignals are discussed hereafter.

Detection of physical activity (physical exertion and lack of physical activity):

Physical exertion (201), i.e., an episode of intense physical activity due to manual labour or physical exercise, may be considered when average metabolic equivalents exceed 6 metabolic equivalents of task (METs) over a predefined time interval, e.g., tens of seconds, few minutes, tens of minutes. Lack of physical activity, i.e., a time interval of sedentary behaviour, may be considered when average metabolic equivalents are below 1.5 METs over a predefined time interval, e.g., a few hours, days, weeks, or a few months. Metabolic equivalents may be estimated from heart rate, obtained from PPG (209) or ECG (210), as 1.05 + 0.11 x HRR, where HRR is heart rate reserve, expressed as

$$HRR = \frac{\text{Heart rate during physical activity - Resting heart rate}}{\text{Predicted maximum heart rate - Resting heart rate}} \text{ x } 100\%,$$

where age-depended predicted maximum heart rate, estimated as 210 - *Age* x 0.79 for woman and 221 - *Age* x 0.95 for men. Resting heart rate is found as an average heart rate during the night since no major activity is expected at this time period. The onset of the night may be set when the number of steps per hour decreases to less than 20. Similarly, the end of the night may be set when the number of steps per hour exceeds 20. The night periods during which the number of steps exceeds 20 may be excluded from the computation of resting heart rate. *HRR* is 0% during rest, whereas it approaches 100% for maximal physical workload.

Alternatively, or in combination, physical exertion may be detected in acceleration signals (207) as the number of steps per time interval. Physical exertion may be considered when the number of steps exceeds 130 steps per minute over a predefined time interval, e.g., tens of seconds, few minutes, tens of minutes. Meanwhile, sedentary behaviour may be considered when the number of steps is lower than 20 steps per hour over a predefined time interval, e.g., a few hours, days, weeks, or a few months.

Detection of posture changes and lying on the left side:

A sudden change in posture, such as from sitting to standing or vice versa, may cause substantial alterations in physiological systems, for instance, a sudden change in blood pressure, which may lead to the initiation of an AF episode or a cluster of AF episodes. Posture changes (202) may be detected in biomechanical rotation signals (206) obtained using gyroscope. Alternatively, or in combination, sitting to standing, standing to sitting, and lying to sitting may be detected in biomechanical acceleration signals (207) by placing the device with the embedded accelerometer sensor on the waist, chest, wrist, thigh, or ankle. The calculation of the angle to determine the posture may be found by analysing accelerations during the activity under interest compared to the reference interval extracted when standing, e.g., during walking. The posture angle is calculated as

$$\theta = \tan^{-1}\frac{x_A x_R + y_A y_R + z_A z_R}{\sqrt{x_A^2 + y_A^2 + z_A^2}\sqrt{x_R^2 + y_R^2 + z_R^2}},$$

where $X_A$, $Y_A$, and $Z_A$ are the mean values of x-, y-, and z-axes for analysis interval, while $X_R$, $Y_R$, and $Z_R$ are the mean axis values for the reference interval. The angle threshold to detect standing is <10° when a vertical axis (a gravitational force vector) is considered as a reference. Lying down (horizontal position) is detected when the angle is >60°. Sitting may be considered when the angle is between 10° and 60°. Horizontal posture may be detected in acceleration signals (207) of lateral x-, vertical y-, and horizontal z-axes. By placing the sensor on the chest, the mean absolute value of horizontal z-axis is higher than the mean absolute value of x- or y-axis for lying on the back or front. Meanwhile, the increase of the mean absolute value of lateral x-axis to 1 g (gravitational constant) is characteristic to the lying down on the left side posture.

Detection of sleep disorders:

Sleep disorders (203), such as poor sleep quality, lack of sleep, snoring or obstructive sleep apnea, may contribute to the AF development and initiation of an AF episode or a cluster of episodes.

Sleep quality may be expressed as a sleep quality score by including information on sleep latency, sleep duration, sleep stages, movement-caused restlessness and heart rate variability. Biosignals, such as PPG (209) or ECG (210), may be used to estimate heart rate, heart rate variability, and respiratory rate. Meanwhile, rotation (206) and acceleration (207) may be used to detect body movements.

Snoring is an audible sign of increased upper airway resistance, detectable in an acoustic signal (208) using non-contact microphones, e.g., those embedded in smartphones. The snoring profile, e.g., intensity levels, frequency ranges, energy peak frequencies, may be quantified in time-domain, e.g., with respect to increasing signal RMS, or by analysing the acoustic power spectrum. The strongest energy in the frequency range from 0 to 20 Hz as well as energy peaks at approximately 100, 400, 600, and 1100 Hz may be considered inherent to snoring.

An episode of obstructive sleep apnea is often accompanied by a decrease in blood oxygen saturation, e.g., below 90%, which can be estimated from PPGs (209) acquired at red and infrared wavelengths. An episode of obstructive sleep apnea may also be accompanied by cyclic variations in heart rhythm obtained from PPG (209) or ECG (210) e.g., a slower heart rhythm (bradycardia) during apnea followed by a faster heart rhythm (tachycardia) upon the end of the apnea episode.

Alternatively, or in combination, sleep information, such as sleep score, analysis of sleep cycles, interruptions in breathing patterns, detection of snoring and obstructive sleep apnea, may be obtained by using sleep tracking mats such as Withings Sleep Analyser (Withings, Issy-les-Moulineaux, France) or Beddit Sleep Monitor (Apple, Cupertino, CA, USA).

Detection of acute emotional stress

Acute emotional stress or anxiety increases heart rate, elevates blood pressure and causes stronger heart contractions due to the release of stress hormones (e.g., adrenaline, noradrenaline, cortisol), therefore it may trigger various heart rhythm disorders, including AF episodes.

Acute emotional stress (204) may be detected by analysing heart rate, heart rate variability, bioimpedance (212), skin temperature (211), and PPG (209) waveform morphology. Acute emotional stress may be detected in heart rate series when heart rate suddenly elevates by about 20% above the baseline resting heart rate and/or heart rate variability reduces by about 20% below the baseline heart rate variability with no apparent physical activity present as measured with an accelerometer (207). Heart rate variability may be estimated relying on conventional time-domain indexes (e.g., RMSSD, SDNN, SDANN, SDSD, NN50, pNN50), frequency domain indexes (e.g., power spectrum density of different frequency bands, frequency power in the 0.15-0.40 Hz range (HF), frequency power in the 0.04-0.15 Hz range (LF), LF/HF ratio), and nonlinear indexes (e.g., Poincare plot of time intervals between successive heart contractions, approximate entropy, sample entropy, correlation dimension, recurrence plot). Baseline heart rate and heart rate variability may be obtained from the rest periods when no movement is detected for a particular time interval.

Alternatively, or in combination, acute emotional stress may be detected in electrodermal activity, a subset of bioimpedance (212), which is the resistance of the skin to a small low-frequency electrical current applied through two electrodes. By analysing electrodermal activity, various parameters, such as the amplitude skin conductance response or integrated skin conductance response, may be calculated. The episode of acute emotional stress may be detected when the aforementioned parameters exceed 50% of baseline values.

Alternatively, or in combination, acute emotional stress may be detected in skin temperature signal (211), e.g., when skin temperature (211) suddenly drops by 0.1 or 0.2 Celsius due to sympathetically mediated vasoconstriction.

Alternatively, or in combination, acute emotional stress may be detected by analysing the morphology of PPG signal (209) waveform, e.g., the amplitude of the forward pulse wave, the duration of diastole, and the time delay of the reflected pulse wave, the angles of the systolic and diastolic slopes.

Detection of dehydration:

Hydration (tissue water content) (205) may be characterized by measuring tissue bioimpedance (212), which depends on tissue resistance to electric current. A decrease in hydration corresponds to an increase in skin impedance due to a reduction of conductive material - water.

Multi-frequency bioimpedance spectroscopy may be used to assess hydration levels. Bioimpedance spectroscopy may involve several different current frequencies, such as from a range from 1 kHz to 1 MHz, to obtain comprehensive information about tissue properties. The measurement of the clamping force of bioimpedance electrodes may be used to ensure stable clamping force of the electrodes and equalize measurement conditions to increase the resolution of hydration levels. Polynomial modelling of limited phase-sensitive multi-frequency measurements of impedance and reactance may be used to draw a dielectric Cole-Cole plot and estimate total body water, intracellular water, and extracellular water. Dehydration level depends on the total body water deficit, e.g., mild dehydration 1-5% of body weight deficit, moderate - 5-10%, strong - >10%.

Biosignal-based detection of AF triggers is not restricted to the use of expert-crafted algorithms as described. Machine learning-based algorithms may also be employed to extract features and identify patterns in biosignals inherent to specific AF triggers.

Machine learning algorithms such as deep learning convolutional neural networks, echo state network, bidirectional long short-term memory networks, and the like, may be trained to detect AF trigger-induced alterations in biosignals. For instance, posture and lying down on the left side may be detected in acceleration (207) signals, obstructive sleep apnea in blood oxygen saturation, respiratory rate, breathing depth, and heart rate series, acute emotional stress in heart rate series, electrodermal activity and temperature (211), dehydration in bioimpedance (212) spectroscopy data, etc.

By combining more than one biosignal, each signal may require a separate neural network for feature extraction, e.g., convolutional neural network, bidirectional long short-term memory, etc., depending on the biosignal itself. The output from the separate neural networks may be aggregated to a shared feature layer and routed to, e.g., multilayer perceptron, support vector machine or another type of a classifier. There might be another neural network, e.g., long short-term memory in between the shared feature layer and the classification layer.

Table 1 below summarizes thresholds for the detection of AF triggers in biosignals.

TABLE 1

| No | Detectable AF trigger | Biosignal | Parameter | Threshold |
|---|---|---|---|---|
| 1. | Physical exertion | ECG, PPG | METs | > 6 METs |
| | | Acceleration | Steps | >130 steps/min |
| 2. | Lack of physical activity | ECG, PPG | MET | <1.5 METs |
| | | Acceleration | Steps | <20 steps/h |
| 3. | Sitting to standing | Acceleration | Posture angle | Angle change from 10°-60° to <10° |
| 4. | Standing to sitting | Acceleration | Posture angle | Angle change from <10° to 10°-60° |
| 5. | Lying to sitting | Acceleration | Posture angle | Angle change from >60° to 10°-60° |
| 6. | Lying on left side | Acceleration | Mean absolute value of x-axis | Increase in mean absolute value to 1 g |
| 7. | Snoring | Acoustic | Spectral energy | Energy peak in the range 0-20 Hz; Energy peaks at 100, 400, 600, 1100 Hz |
| 8. | Obstructive sleep apnea | PPG | Blood oxygen saturation | <90% |
| | | PPG, ECG | Heart rhythm | Cyclic variations in heart rhythm |
| 9. | Acute emotional stress | PPG, ECG | Heart rhythm | Increase by >20%, decrease in heart rate variability by >20% |
| | | Electrodermal activity | Skin conductance response | >50% of baseline value |

(continued)

| No | Detectable AF trigger | Biosignal | Parameter | Threshold |
|----|----|----|----|----|
| | | | Integrated skin conductance response | >50% of baseline value |
| 10. | Dehydration | Temperature | Skin temperature | Sudden drop by >0.1 °C |
| | | Bioimpedance | Total body water deficit | Mild 1-5% of body weight deficit |
| | | | | Moderate 5-10% of body weight deficit |
| | | | | Strong >10% of body weight deficit |

Collection of person-reported AF triggers will be discussed hereafter. A module configured to collect information about reported AF triggers (102) may be implemented as a software application in a local computing device such as a smartwatch, a smartphone, a tablet, a laptop, a personal computer. Examples of reported AF triggers may include alcohol, caffeine, acute emotional stress, anxiety, large meals, cold foods and beverages, high salt intake, certain foods (e.g., onions, nuts, chocolate, ice cream, spicy food, garlic), bloating, constipation, emesis, relaxing, tiredness, infections, etc. The reported AF triggers are time-stamped so that the triggers can be matched with the temporal AF episode occurrence pattern.

The module may be configured to interact with one or more biosensors or medical devices to complement some of the person-reported triggers with objective information extracted from biosignals. For instance, reported acute emotional stress and anxiety may be accompanied by detected changes in heart rate variability, skin temperature or conductance. Signs and symptoms of sleep disorders may be accompanied by information such as sleep cycles, sleep quality, snoring / apnea detection, and the like, provided by smart sleep trackers.

The dashboard of a user interface (300a) for collecting reported AF triggers may enable entering suspected triggers (301), e.g., by selecting from the given list (302) or entering a new suspected trigger (303) in case the suspected trigger is not included in the list. The dashboard may also allow sending messages about the observed signs and symptoms related to AF and may also display recommendations to avoid specific triggers for a particular time interval, e.g., several weeks or months. Recommendations may be generated either automatically or entered manually by a medical staff member.

The dashboard of a medical staff member interface (300b) for analysing person's information may include data on timestamped AF triggers and the AF episode occurrence pattern (304), AF episode pattern-characterizing parameters (305), reported (306) and detected (307) triggers with the calculated causality score (308) between each suspected trigger and the AF episode occurrence pattern. The dashboard of a medical staff member interface (300b) may also provide information on the risk of dangerous diseases, e.g., stroke, heart failure, as well as allow entering and sending personal recommendations for the user.

Transformation of triggers to time-series signals will be discussed hereafter. The aforementioned biosignal-based triggers are detected in time-series signals, therefore can be directly matched with the temporal AF episode occurrence pattern for the purpose of calculating the causality score between the detected AF triggers and the AF episode occurrence pattern.

Meanwhile, the reported person-entered triggers, such as alcohol, caffeine, reported acute emotional stress or anxiety, large meals, cold beverages, cold foods must be transformed (103) from the timestamped data to time-series signals to establish the causality score. The unit-amplitude pulses, starting at the time instances the triggers are timestamped by the person, are characterized by the growth function (e.g., exponential, bounded exponential, linear) with the growth rate parameter $\alpha_0$ and by the decay function (e.g., exponential, bounded exponential, linear) with the decay rate parameter $\alpha$. The growth function and the parameter $\alpha_0$, as well as the decay function and the parameter $\alpha$ are determined specifically for each AF trigger based on the clinically established properties. The signal outside the transformed AF triggers may be filled with determined baseline values, which depend on the long-term adherence of the person to the trigger, or with zeros if such information is not available.

Transformation of alcohol consumption to body reactivity signal:

Alcohol consumption may be expressed in terms of alcohol units where a glass (25 ml) of strong alcohol (e.g., 40% vodka) is about one alcohol unit, a glass (200 ml) of medium alcohol (10% wine) contains about two alcohol units, a bottle (500 ml) of weak alcohol (e.g., 5% beer) contains about three alcohol units. The average absorption rate of alcohol for an average person weighting 70 kg is about 10 grams per hour. Therefore, the absorption rate may be characterized by a bounded exponential growth function defined as $y(x) = \beta(1-\exp(\alpha_0 \cdot x))$, where $\beta$ is an alcohol unit (i.e., 2 units for a glass of wine), while $\alpha_0$ is the growth parameter set to -10.6/$\beta$ units/h (401). The amplitude of alcohol absorption is determined by the number of alcohol units. Various factors may affect alcohol absorption, e.g., eating fat food may slow down, whereas drinking carbonated beverages may speed up absorption. Meanwhile, the metabolic capacity to remove alcohol is about 7 grams per hour which translates to about one alcohol unit per hour. Alcohol removal may be characterized by a linear function with a decay parameter $\alpha$ set to 1 unit/h (402).

Considering that women have less body water than men of similar weight and have fewer enzymes to metabolize alcohol, women often have higher alcohol concentrations after drinking equivalent units of alcohol. Also, persons who weigh more are more likely to have a lower blood alcohol concentration than those weighing less. Therefore, gender information and body mass index (BMI) may be included to adjust the parameters of alcohol consumption-characterizing functions.

Transformation of caffeine consumption to body reactivity signal:

Caffeine consumption may be expressed in terms of coffee cups (e.g., standard 12-oz cup, 355 ml). Following ingestion, caffeine is rapidly absorbed reaching peak concentration in the circulation within one hour. Given that the standard cup of coffee contains 140 mg of caffeine, the absorption rate may be characterized by a bounded exponential growth function, where $\beta$ is cups of coffee, with the growth parameter $\alpha_0$ set to -6.9 cups/h (403). Meanwhile, the average caffeine elimination rate is about 40 mg per hour and may be characterized by an exponential decay function with a decay parameter $\alpha$ set to -0.6 cups/h (404).

Transformation of large meal consumption to body reactivity signal:

Large meal intake may be expressed in terms of energy production and digestion time. Following ingestion of a large meal, energy production increases up to a maximum level, which depends on the meal type and size. The maximum level can be defined based on food energy (kJ) and digestion time (h). The large meal (e.g., beef, pork, fried fish) provides about 1000 kJ and takes about 5 h to digest, resulting in the maximum level of 200 kJ/h. Increase in energy production can be characterized by a bounded exponential growth function, where $\beta$ is the maximum level set to 200 kJ/h, while $\alpha_0$ is the growth parameter set to -3.8 kJ/h (405). Digestion starts after 2 h and may be characterized by a linear function with a decay parameter $\alpha$ set to -40 kJ/h (406).

Transformation of cold beverage and food consumption to body reactivity signal:

Cold beverages and foods may stimulate the parasympathetic system due to decreased intragastric temperature and thus contribute to the initiation of AF episode or a cluster of episodes. Intragastric temperature, which is about 37 °C, drops to about 22 °C after ingestions of 400 ml of cold drink (about 4 °C) and returns to body temperature within 30 minutes. Decrease in temperature may be characterized by a linear function with the decay parameter set to -15 °C/min (407). The return to body temperature may be characterized by a bounded exponential growth, where $\beta$ is intragastric temperature, i.e., 37 °C, with the growth parameter set to -0.2 °C/min (408).

Transformation of reported acute emotional stress or anxiety to body reactivity signal:

During the episode of acute emotional stress or anxiety, the sympathetic system is activated to the "fight or flight" response which results in increase in skin conductance due to sweat production and increase in heart rate. Body's response to the acute emotional stress may be characterized by a bounded exponential growth function, where $\beta$ is stress units, with the growth parameter $\alpha_0$ set to -1.1 stress units/min (409). Meanwhile, body recovery from stress may be characterized by an exponential decay function with a decay parameter $\alpha$ set to -0.3 stress units/min (410).

Table 2 below summarizes functions and parameters required to transform reported triggers to the time-series multivariate signals.

TABLE 2

| No | Reported trigger | Growth function, growth parameter $\alpha_0$ | Decay function, decay parameter $\alpha$ |
|---|---|---|---|
| 1. | Alcohol | Bounded exponential, -10.6/$\beta$ units/h, where $\beta$ is an alcohol unit | Linear, 1 unit/h |
| 2. | Caffeine | Bounded exponential, -6.9 cups/h, where $\beta$ is cups of coffee | Exponential, -0.6 cups/h |
| 3. | Large meals | Bounded exponential, -3.8 kJ/h, where $\beta$ is maximum energy production level | Linear, -40 kJ/h |

(continued)

| No | Reported trigger | Growth function, growth parameter $\alpha_0$ | Decay function, decay parameter $\alpha$ |
|---|---|---|---|
| 4. | Cold beverages and foods | Linear, -15 °C/min | Bounded exponential, -0.2°C/min, where $\beta$ is intragastric temperature |
| 5. | Acute stress and anxiety | Bounded exponential, -1.1 stress units/min, where $\beta$ is stress units | Exponential, -0.3 stress units/min |

Collection of temporal AF occurrence patterns will be discussed hereafter. A temporal AF episode occurrence pattern (501) may be defined as a temporal distribution of self-terminating AF episodes over a predefined time period, e.g., days, weeks, months, years. AF episodes (502), with beginning defined as a transition from normal or other heart rhythm to AF and the end defined as a transition from AF to normal or other rhythm, may distribute sporadically over the monitoring period or manifest in clusters.

A module configured to collect temporal AF episode occurrence patterns (104) may consist of one or more integrated biosensors to acquire biosignals. Biosignals are then analysed for AF in real-time or offline with a dedicated AF detection algorithm. For example, AF occurrence patterns may be collected using a biooptical PPG sensor (503) which operates in combination with a biopotential ECG sensor (504). Initial temporal AF episode occurrence patterns may be collected by analysing solely PPG signal, whereas heart rhythm change to AF and vice versa may be confirmed or rejected relying on the short-term ECG. An alert notification (505) to acquire an ECG (506) may appear after detection of AF episode in PPG signal or at specific time intervals. An alert notification (505) may be implemented as vibration of the module, beep, or blinking light. The module with the embedded sensors may be a wrist-worn device, such as a smartwatch, a bracelet, and the like. Alternatively, temporal AF episode occurrence patterns may be collected using external ECG recorders, such as Holter monitors and ECG patches, or implantable devices capable of analysing intra-atrial electrogram acquired directly from the heart via an atrial lead.

The ECG characteristics inherent to AF, e.g., heart rhythm irregularity, the absence of normal atrial activity-representing P waves and the presence of abnormal atrial activity-representing f-waves, may be analysed by an automatic algorithm. For instance, heart rhythm irregularity may be quantified using statistical dispersion measures (e.g., coefficient of variation, number of turning points, histogram-based parameters), entropy (e.g., Shannon entropy, sample entropy, simplified entropy), symbolic dynamics, Poincare plot, and time-varying coherence function. P wave absence may be detected by characterizing P wave morphology (e.g., P wave amplitude in contiguous 20 ms intervals, PR interval variance, skewness, kurtosis, fit of Gaussian function to the P wave). The presence of f-waves may be analysed by applying f-wave extraction techniques (e.g., average beat subtraction, spatiotemporal beat subtraction, echo state neural network) and analysing the obtained signal with respect to spectral properties (e.g., spectral concentration, peak frequency in 3-12 Hz range, spectral entropy). Meanwhile, the cardiac specialist may access the ECG through the remote server or cloud service.

Characterization of AF episode occurrence patterns will be discussed hereafter. One way to identify a causal link between the suspected AF triggers and the AF episode occurrence pattern is to analyse the temporal distribution of suspected AF triggers and AF episode occurrence pattern. However, AF episodes may manifest in clusters or aggregate within a specific time interval (e.g., during the night), therefore, patterns may be translated to pattern-characterizing parameters, e.g., that account to AF episode clustering or aggregation over time, to provide additional information on the influence of a specific trigger on the occurrence of AF episodes.

Temporal AF episode occurrence patterns may be characterized with respect to AF burden (601), which is the proportion of time an individual is in AF compared to the total monitoring period. AF burden may be expressed from 0 to 1, where 0 stands for no AF during the entire monitoring period, whereas 1 is obtained for AF taking the entire monitoring duration.

Alternatively, or in combination, temporal AF occurrence patterns may be characterized using the duration of the longest AF episode (602) or the number of AF episodes (603) during a predefined monitoring period, e.g., 1 day, 3 days, 1 week, 2 weeks, 1 month, 3 months, 6 months, 1 year.

Alternatively, or in combination, temporal AF occurrence patterns may be characterized using inequality indexes (604), such as the Gini coefficient, which approaches values close to 0 for the uniform distribution when AF episodes are of similar duration, whereas approaches values close to 1 for widely different episode durations.

Alternatively, or in combination, temporal AF occurrence patterns may be characterized using temporal AF episode concentration indexes (605), which take values close to 1 for high temporal aggregation, inherent to patterns with a single short continuous AF episode or a single dense cluster of AF episodes, whereas values close to 0 indicate low temporal concentration, inherent to AF patterns with episodes evenly spread over the monitoring period, e.g., 1 day, 3 days, 1 week, 2 weeks, 1 month, 3 months, 6 months, 1 year.

Alternatively, or in combination, temporal AF occurrence patterns may be characterized using indexes which reflect the clustering (606) of AF episodes, e.g., history-dependent point process modeling may be employed to characterize AF episode clusters using an alternating bivariate Hawkes model. For example, exponential decay rate of conditional intensity function, which specifies the mean number of transitions from AF to non-AF in an interval conditional on the past, is related to episode clustering. That is, low values increase the likelihood that an episode is followed by additional episodes, resulting in a cluster, while an increase of intensity decay rate leads to AF episodes more spread in time.

Matching of AF trigger and pattern data will be discussed hereafter. A software module (106) is configured to match the temporal AF episode occurrence pattern, or some pattern-characterizing parameter, with the suspected AF triggers, either reported by the person or automatically detected in the biosignals. To increase reliability of causality estimation, the data collection should preferably embrace the entire monitoring period.

In case of person-reported triggers (700a), the sample-by-sample signal comprises of the specific time intervals with the transformed suspected AF triggers (701) and the time intervals outside the transformed suspected AF triggers (702). Outside intervals may be filled with zeros (702) or with determined baseline values, which may depend on the long-term adherence of the person to the specific trigger. The temporal AF episode occurrence pattern may comprise of ones (703) during the AF episode and zeros (704) outside the AF episode.

In case of detected triggers (700b), the signal consists of accumulated value within some time interval, e.g., minute, hour, day. For instance, in case of physical exertion, the accumulated signal may comprise of METs per hour. Only the time period when METs exceed a threshold (705) of 6 METs per hour are considered as a detected physical exertion trigger (706). Outside-time intervals consist of accumulated MET values within the predefined time interval (707). To match the accumulated data, the temporal AF episode occurrence pattern should be accumulated as well, e.g., by expressing as an AF burden per hour (708).

Establishing the causality score between the trigger and the temporal AF episode occurrence pattern will be discussed hereafter. A software module (107) is configured to establish the causal relationship between particular suspected AF trigger and the temporal AF episode occurrence pattern and generate the causality score (800). The causality score may be used to show the likelihood that the causal relationship between a particular suspected AF trigger and the AF episode occurrence pattern exists.

The temporal AF episode occurrence pattern is loaded from the personal computing device's memory (801) and quantified using pattern-characterizing parameters (802). During the next step, the timestamped sequence of a particular suspected trigger (e.g., alcohol) is loaded from the personal computing device's memory (803) and transformed to a continuous time-series signal (804). During the next step, the causality score (805) is computed between the time-series signal of a suspected trigger and the temporal AF episode occurrence pattern-characterizing parameter (e.g., AF burden in a time interval of 1 hour). The relationship is considered causal when the causality score exceeds the predefined threshold (806).

The causal relationship may be used to notify (807) the user and the medical staff member about the identified AF trigger via software application implemented in the personal computing device or further computing device. Then, the algorithm for establishing the causality score is repeated (809) for another suspected trigger (e.g., caffeine). The algorithm is repeated until all suspected triggers are analysed with respect to causality with the temporal AF episode occurrence pattern (810).

The time-series representation of suspected triggers and the quantified temporal AF episode occurrence pattern may be analysed using one or more causality analysis techniques, such as Granger causality test. Granger causality is a statistical approach based on prediction, that is, if an independent signal $X$ (i.e., AF trigger time-series) "Granger-causes" a dependent signal $Y$ (i.e., AF episode or a cluster of AF episodes), then trigger occurrence signal $X$ should be useful when predicting the signal $Y$. According to the definition, the $X$ signal "Granger-causes" the $Y$ signal when the prediction of the $Y$ signal is improved by including the past values of the signal $X$. An important aspect of Granger causality is that no instantaneous causation is possible, thus causal response should involve time lags $L$. The optimal lag length for $X$ and $Y$ is determined relying on the Bayesian information criterion where the time lag with the lowest Bayesian information criterion value is chosen. The Granger causality assumes that both the $X$ and $Y$ signals are stationary, thus detrending must be employed before computing Granger causality if the condition of stationarity is unsatisfied. Detrending may weaken the causal relationship, while the lack of detrending may falsely enhance the causal relationship.

The causality score $G$ using the approach of Granger causality may be given as a quantitative value, by using the equation below:

$$G = 1 - p \text{ value,}$$

where $p$ value is the number between 0 and 1, and indicates the probability that causality between X and Y may have occurred due to a random chance assuming that no causality between X and Y exists (the null hypothesis of noncausality). G approaches 1 for a perfectly "Granger-causal" relationship, whereas approaches values close to 0 for a random relationship. A score between 0.99 and 1 may indicate a very strong relationship, between 0.95 and 0.99 a strong relationship, between 0.9 and 0.95 a moderate relationship, whereas a score lower than 0.9 may represent a weak relationship. A causality score is computed for each time-series of suspected AF triggers and the temporal AF occurrence pattern. Out of all causality tests performed for each suspected trigger, only those triggers with G value greater than 0.9 are assumed to be "Granger-causal" for the occurrence of AF episode or a cluster of episodes for a particular number of lags *L*.

The approach for computing the causality score is not limited to the approach of Granger causality. Other techniques for causality analysis, such as causal Bayesian networks or Mendelian randomization causality method, may be used instead.

Examples of use of causality score between AF triggers and AF pattern will be discussed hereafter. The examples serve as means for conveying the applicability of the invention. The listed examples are demonstrative and neither intended to be exhaustive nor comprehensive.

Example 1. Modification of the temporal AF episode occurrence pattern by recommending avoiding identified AF triggers.

A medical staff member may use the proposed invention as nonpharmacological approach to modifying the temporal AF episode occurrence pattern by recommending the person to avoid the identified AF triggers.

Based on the analysis of personally identified AF triggers, the feedback recommendations may be generated automatically or entered manually by a medical staff member.

Upon initiating nonpharmacological treatment, the person is monitored for a particular time interval, e.g., 1 week, 2 weeks, 1 month, until the causality scores for the suspected AF triggers are generated and triggers are identified (108). Onwards, causality scores are updated in defined time intervals, e.g., every week, month, year. Written and/or verbal recommendation (901) about behavioural changes based on individually generated causality scores may be provided to the person by a personal smart device, e.g., a smartwatch, a smartphone, a tablet, a laptop, a personal computer. Written and/or verbal recommendation may include a brief, e.g., a sentence or two, explanation on possible relationship between the trigger and the AF episode. Written and/or verbal recommendation may also include specific suggestion for behavioural change, e.g., "Practice meditation, yoga, progressive muscle relaxation, deep breathing or exercising if you feel stressed", "Lower alcohol consumption", "Go to bed early and get enough sleep", "Avoid sleeping on the left side", "Consult your doctor regarding sleep apnoea", "Take your anti hypertension medication at the same time". The person may also receive written and/or verbal encouragement if the adherence to recommendations results in the improvement in the temporal AF episode occurrence pattern.

Interpretation of temporal AF occurrence patterns. The change in AF episode occurrence pattern (902), as a result of avoidance of individually identified triggers (108), may be assessed based on the parameter values for characterizing temporal AF episode occurrence pattern (105).

The improvement in AF occurrence pattern may be a reduction in AF burden by a certain percentage, e.g., 5%, 10%, 20%, for the same duration of the monitoring period, e.g., 1 day, 3 days, 1 week, 2 weeks, 1 month, 3 months, 6 months, 1 year. Alternatively, or in combination, the improvement in AF occurrence pattern may be a reduction of the duration of the longest AF episode by a certain percentage, e.g., 5%, 10%, 20%, for the same duration of the monitoring period. Alternatively, or in combination, the improvement in AF occurrence pattern may be a reduction of the number of AF episodes by a certain percentage, e.g., 5%, 10%, 20%, for the same duration of the monitoring period. Alternatively, or in combination, the improvement in AF occurrence pattern may be a reduction in aggregation by a certain percentage, e.g., 5%, 10%, 20%, for the same duration of the monitoring period. Alternatively, or in combination, the improvement in AF occurrence pattern may be a reduction in clustering by a certain percentage, e.g., 5%, 10%, 20%, for the same duration of the monitoring period.

Table 3 below provides an example of the guiding rules for interpretation of the temporal AF episode occurrence pattern.

TABLE 3

| No | Parameter | Monitoring period | Interpretation | |
|---|---|---|---|---|
| | | | Improvement | Deterioration |
| 1 | AF burden | 1 week | Decrease by >5% | Increase by >5% |
| 2 | Duration of the longest episode | 1 week | Decrease by >10% | Increase by >10% |
| 3 | Number of AF episodes | 1 week | Decrease by >20% | Increase by >20% |
| 4 | Aggregation | 1 week | Decrease by >5% | Increase by >5% |
| 5 | Clustering | 1 week | Decrease by >5% | Increase by >5% |

Example 2. Identification of a pathological chain of events leading to AF episode occurrence.

Specific triggers which provoke AF for persons with particular risk factors, as well as physiological mechanisms underlying these triggers, are not yet understood. Trigger-induced alterations in physiological systems, especially those modulating atrial electrophysiology, may play an important role in the initiation and progression of self-terminating AF. Hence, understanding trigger-induced repetitive alterations in physiological systems is of importance when protecting atria from further remodelling.

A medical staff member may use the proposed invention to identify a pathological chain of events leading to AF occurrence. Joint analysis of existing risk factors (e.g., obesity, heart failure) (1001), echocardiography parameters (e.g., left atrial size, left atrial strain) (1002), trigger-induced alterations in physiological systems (e.g., autonomic nervous system, blood pressure) (1003) together with the identified AF triggers (108) may facilitate understanding of a pathological chain of events (1004) leading to AF for a particular person. This may suggest potential treatment targets (1005) to interrupt the pathogenetic chain, ultimately resulting in fewer AF episodes, less AF episode aggregation, or clustering.

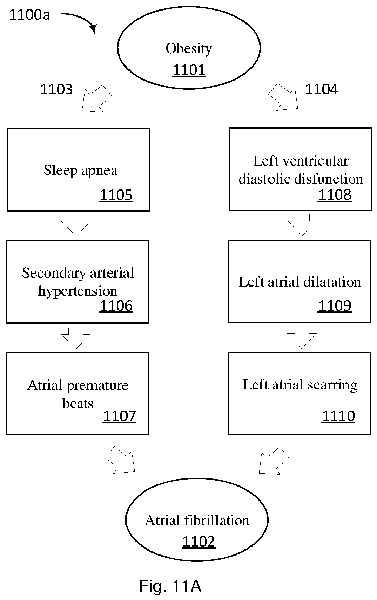

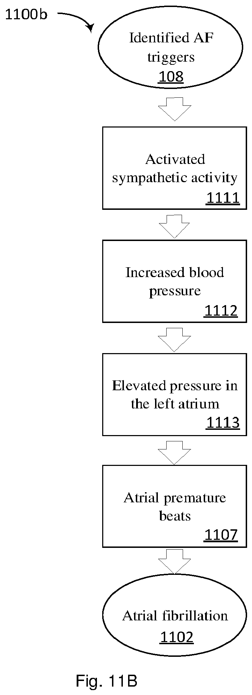

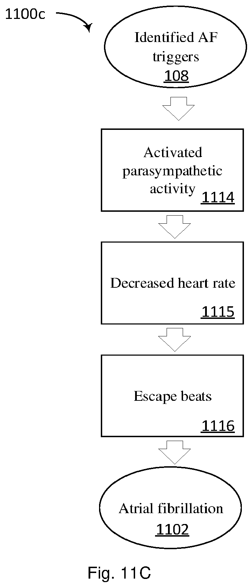

An example of such a pathological chain of events (1100a), potentially identifiable using the embodiment of the invention, in patients with obesity (1101) is given in FIG. 11. Two cooperating pathways that are essential for AF occurrence (1102) may be identified in obese individuals (body mass index >30 kg/m$^2$). The first path (1103) relates to transient AF triggers which may induce AF, whereas the second path (1104) relates to gradual structural changes of the left atrium creating substrate to sustain AF after induction. Obesity may cause sleep disturbances, such as episodes of obstructive apnea (1105), which can be captured using sleep analysis mat. Sleep disturbances may cause secondary hypertension (1106), observed by blood pressure monitoring. The latter provokes premature atrial beats (1107) from pulmonary veins of the left atrium which may be detected in ECG or PPG signals recorded using standard Holter monitoring, ECG patches, smartwatches, and the like. Obesity (1101) may also result in diastolic disfunction of the left ventricle (1108) leading to the left atrial dilatation (1109), as seen in the echocardiography image (left atrial size >5.6 x 4.6 cm or left atrial volume index >40 ml/m$^2$). This causes left atrial scarring (1110) which manifest in slow conduction zones with a favourable substrate for sustaining AF as well as increasing AF burden, aggregation, and clustering.

Trigger-induced activation of sympathetic activity. Another example of such a pathological chain of events (1100b) involves the mechanism of the activated sympathetic component of the ANS (1111), which results in increased systemic blood pressure (1112) and elevated pressure in the left atrium (1113). Sympathetic activity may be stimulated by some triggers such as caffeine, emotional stress, excessive physical activity, dehydration. Pressure in the left atrium may initiate runs of atrial premature beats (1107) leading to AF occurrence (1102). Activation of the mechanism of increased sympathetic activity (1111) may be identified as an increase in blood pressure and/or increase in the number of atrial premature contractions, the occurrence of atrial bigeminy, trigeminy, quadrigeminy, couplets, triplets, and atrial runs.

Trigger-induced activation of parasympathetic activity. Yet another example of such a pathological chain of events (1100c) involves the mechanism of the activated parasympathetic component of the ANS (1114), which results in a decreased heart rate (1115), and thus, provoke atrial premature beats (1107) or escape heartbeats (1116). Parasympathetic activity may be stimulated by some triggers such as cold food, cold beverages, certain alcohol (e.g., red wine and strong alcohol), sleep, resting. Activation of the mechanism of increased parasympathetic activity (1114) may be identified as an increase in the number of atrial premature contractions and escape heartbeats.

Accounting to risk factors which may influence the susceptibility to AF triggers. Susceptibility to AF triggers may depend on existing long-term risk factors which may cause impairment in left ventricular diastolic function, resulting in the left atrium fibrosis and scarred tissue. Scars induce zones of slow conductivity and lead to abnormal conduction through the atria, creating a substrate for AF. Examples of risk factors to account for when assessing the susceptibility to AF triggers are age, hypertension, obesity (BMI > 30 kg/m$^2$), obstructive sleep apnea, physical inactivity, excessive physical activity, excessive alcohol consumption, hyperlipidemia, smoking, diabetes, renal dysfunction, genetic predisposition, hyperthyroidism, increased adrenal function, heart failure, coronary heart disease.

Accounting to existing damage in the atrial tissue. The existing damage in the atrial tissue may be characterized using echocardiography parameters (1002) with the following approximate abnormal threshold values, such as increased left atrial size (thickness female >3.8 cm, male >4.0 cm, dimensions >5.6 x 4.6 cm), abnormal left atrial strain, slow left atrial appendage velocity (<0.5 m/s), high left atrial volume index (>40 ml/m$^2$), abnormal diastolic function of the left ventricle, decreased left ventricular ejection fraction (<50%).

Detection of atrial premature beats and related rhythms. Atrial premature beats may be detected in the heart rate series, expressed as RR interval series (RR interval in seconds = 60/heart rate in beats per min). RR intervals may be extracted from an ECG (210) or PPG (209). Alternatively, RR intervals may be extracted from intra-atrial or intraventricular signals acquired using implantable devices. Atrial premature contractions with reset of the sinus node are detected when the sum of the length of the preceding and the subsequent RR intervals is less than twice the normal RR interval. Atrial premature contractions with delayed reset of the sinus node may be detected when the preceding RR interval is shorter by approximately 20% and subsequent RR interval is prolonged by approximately 20% compared to the normal RR interval. Premature atrial contractions with full compensatory pause may be detected when the subsequent RR interval is equal to twice the preceding RR interval. Atrial premature contractions manifesting in patterns may be classified as bigeminy (every other beat is premature), trigeminy (every third beat is premature), quadrigeminy (every fourth beat is premature). Couplets, triplets, and atrial runs may be detected as two, three and four or more consecutive atrial premature beats, respectively.

Detection of trigger-induced alterations in the ANS. Trigger-induced alterations in the ANS may be characterized using ANS indexes, which may be calculated from RR intervals, extracted from one or more biosignals, such as PPG (209), ECG (210), in relation to physical activity signals such as rotation (206), acceleration (207), altitude.

Post-exercise heart rate recovery is non-invasive approach to assess cardiovascular autonomic function by measuring how quickly heart rate returns to normal after intensive physical exercise (1200). Physical activity, such as fast walking or stair climbing, may be expressed as a number of steps per minute (1201). Heart rate recovery in heart rate series may be approximated by a mono-exponential model (1202) by fitting it in a 5 min time interval after the physical activity. Then the time-constant $T$ (1203) of exponential decay may be estimated. The quality of exponential fitting may be assessed via the coefficient of determination which should exceed a fixed threshold of 0.5 to consider the fitting acceptable.

The rapid heart rate decay immediately after the physical activity may be characterized by the short-term time constant T30 (1204), found by fitting a line to the logarithm of heart rate and being the negative inverse of the slope of the resulting line (1205). The decay of heart rate in 30 s (HRR30) (1206), 60 s (HRR60) (1207) and 120 s (HRR120) (1208) after the physical activity, and the difference between the maximal heart rate at recovery onset and the baseline heart rate (ΔHR) (1209), i.e., minimal heart rate at the end of the recovery period, may also be estimated.

Detection of trigger-induced alterations in arterial blood pressure. Trigger-induced alterations in a cardiovascular system may be characterized by arterial blood pressure parameters such as systolic, diastolic, mean arterial pressure, pulse pressure, i.e., the difference between systolic and diastolic blood pressure. Change in arterial blood pressure may be detected by finding pulse arrival time or pulse transit time which depends on the pulse wave delay when propagating from one part of the body to the other part, e.g., from the heart to the ear, toe, wrist, finger. The pulse delay time depends on the vascular tone which increases with increasing blood pressure.

The pulse arrival time (1301) may be estimated using a combination of ECG (1302) and PPG (1303) signals. In the ECG, the fiducial point representing ventricular contraction (e.g., R peak) is taken as the proximal reference point. In the PPG, various distal reference points are common, such as foot or maximum of the pulse wave, maximum of the first or second derivative, the intersection of tangent lines to the steepest upstroke segment and the foot.

The pulse transit time (1304) may be estimated from two PPG signals recorded at different body locations, one being proximal (1305) and another being distal (1306) with respect to the heart. The reference points of pulse waves of both PPG signals for pulse transit time estimation can be the same as in case of pulse arrival time estimation, e.g., foot or maximum of the pulse wave, maximum of the first or second derivative, the intersection of tangent lines to the steepest upstroke segment and the foot. Alternatively, the pulse transit time may be estimated from a waveform of the PPG signal by decomposing pulse wave into forward (1307) and backward (1308) waves and estimating the difference in their occurrence times (1309). Since the time taken for the pulse wave to travel the arterial path depends on the path's length, measurement of the distance between the heart and the PPG sensor or between the two PPG sensors or their approximation based on subject's height should be considered.

Changes in pulse arrival time or pulse transit time could be related to changes in blood pressure, obtained using a reference blood pressure measurement (1310), for individual subject through initial calibration (1311) using a nonlinear model

$$BP = T/K1 + K2,$$

where T is pulse arrival time or pulse transit time, and K1 and K2 are calibration constants. To maintain accuracy, automatic recalibration may be performed every several minutes, every several hours, once a day, once a week, etc.

In another embodiment, different features obtained by analysing pulse wave of PPG signal may be employed for estimating changes in arterial blood pressure. These include, e.g., pulse rising time, pulse area, pulse width at different amplitude percentage, the ratio of systolic and diastolic peak amplitudes. Since these features are not directly related to pulse delay time, their relation to blood pressure changes may be established through nonlinear machine learning-based models and maintained through recalibration.

Example 3. Personal assessment of ischemic brain stroke risk.

Stroke risk assessment based on existing risk factors and AF occurrence pattern. In general, the risk of ischemic brain stroke is assessed relying on the existence of long-term AF risk factors, such as obesity, hypertension, diabetes mellitus, coronary artery disease, and heart failure. These AF risk factors contribute to AF development due to induced electrical and structural changes in the atria that create favourable conditions for AF initiation and sustenance. The $CHA_2DS_2$-VASc stroke score is often used for the assessment of stroke risk in individuals with AF. Accounting for individual-specific factors, such as the temporal AF episode pattern and the morphology type of the left atrial appendage, may increase the accuracy of stroke risk assessment.

The identified AF triggers (108) followed by personal recommendations to avoid identified triggers (901) may modify the temporal AF episode occurrence pattern (104), resulting in changed pattern characteristics (105). A medical staff member may use the information on the temporal AF episode occurrence pattern to assess the risk of brain stroke (1401). The personal stroke risk score may also comprise of existing risk factors (1402) and morphology type of left atrial appendage (1403). Based on the personal stroke risk score, clinical treatment may be modified (1404), e.g., by reconsidering the dosage of blood-thinning medication.

Blood emptying velocity, i.e., maximal flow velocity, in left atrial appendage is a risk factor for thrombus formation and depends on the morphology of left atrial appendage (e.g., chicken wing, cactus, windsock, cauliflower). Maximal emptying flow velocity in AF patients with left atrial appendage morphology of chicken wing or windsock is larger comparing to patients with left atrial appendage morphology of cactus or cauliflower.

Morphology type of left atrial appendage may be determined by contrast-enhanced electrocardiogram-gated computed tomography or contrast-enhanced magnetic resonance imaging. These imaging techniques also enable computation of standard measurements such as left atrial appendage volume and blood emptying velocity.

The personal stroke risk score may be determined by combining the information of well-established $CHA_2DS_2$-VASc score, which rely on existing risk factors, i.e., congestive heart failure, hypertension, age $\geq$75, diabetes mellitus, prior stroke or transient ischemic attack, vascular disease, age 65-74, female (1402), together with the morphology type of left atrial appendage (1403) and the characteristics (105) of the temporal AF episode occurrence pattern (104):

$$\textit{Personal stroke risk score} = \textit{CHA}_2\textit{DS}_2\textit{-VASc score} + \textit{Morphology} + \textit{AF pattern,}$$

where *Morphology* - a weight coefficient for morphology type of left atrial appendage, and *AF pattern* - weight coefficient for temporal AF episode occurrence pattern. The maximum personal stroke risk score is 12.

$CHA_2DS_2$-VASc may be set from 0 to 9, i.e., 1 for congestive heart failure, 1 for hypertension, 2 for age $\geq$75, 1 for diabetes mellitus, 2 for prior stroke or transient ischemic attack, 1 for vascular disease, 1 for age 65-74, and 1 for female.

*Morphology* may be set to 2 for left atrial appendage morphology of cauliflower, set to 1 for cactus, and set to 0 for windsock and chicken wing.

*AF pattern* may be set to either 0 or 1. *AF pattern* may be set to 1 if the daily AF burden is more than 5.5h, or if AF episodes are aggregated (aggregation index >0.5), or if AF episodes are clustered (clustering index <0.03). Otherwise, *AF pattern* is set to 0.

## Claims

1. Method for establishing a causality score between atrial fibrillation triggers and atrial fibrillation occurrence pattern for management of personally identified atrial fibrillation triggers in relation to temporal atrial fibrillation occurrence patterns, the method steps being carried out by a software of a personal computing device and server wherein the method steps comprise:

   - detecting (101) AF trigger occurrence instance in biosignals,
   - enrolling (102) data representing reported AF triggers,
   - transforming (103) the enrolled data representing AF triggers to time-series signals,
   - constructing (104) temporal AF episode occurrence patterns,
   - characterizing (105) temporal AF episode occurrence patterns with respect to episode aggregation or/and clustering,
   - matching (106) AF trigger and temporal AF episode occurrence pattern data,
   - calculating (107) the causality score between the suspected AF triggers and the temporal AF episode occurrence pattern for identifying personal AF triggers,

   where data of biosignal information is assigned a threshold value depending on biosignal information source,
   where data of biosignal information corresponding to said threshold value or exceeding said threshold value is assigned to a particular trigger,
where data of reported triggers is transformed to time-series signals starting at the time a trigger is timestamped **characterized by** the growth function with the said growth rate value and by the decay function with the said decay rate value,
where the collected AF episode occurrence instances, as a function of time from data of biosignal information corresponding to said threshold value or exceeding said threshold value, form a temporal AF episode occurrence pattern supplemented with weight value with respect to increase or decrease values of burden, concentration, number of occurrence instances, clustering of occurrence instances, longest occurrence instance, shortest occurrence instance, occurrence instance inequality,
where the causality score is calculated using causality estimation test between the time-series signal of a suspected trigger and the temporal AF episode occurrence pattern-characterizing parameter.

2. Method according to claim 1, where the causality estimation test is Granger causality test or causal Bayesian networks or Mendelian randomization causality method.

3. Method according to any one of 1-2 claims, where the data of biosignal information comprises data of signals such as PPG, ECG, acceleration, rotation, altitude, acoustic, body temperature, bioimpedance, blood pressure, respiration.

4. Method according to any one of 1-3 claims, where the causality score is a quantitative value from 0 to 1, where the score value between 0.99 and 1 indicates a very strong relationship, the score value between 0.95 and 0.99 indicates a strong relationship, the score value between 0.9 and 0.95 indicates a moderate relationship, whereas the score value lower than 0.9 may represent a weak relationship.

5. Method according to any one of 1-4 claims, where AF trigger occurrence instance data comprises threshold of a value designating an average metabolic equivalent exceeding 6 metabolic equivalents of a task (METs) over a predefined time interval or a value designating an average metabolic equivalent below 1.5 METs, and/or value designating a number of steps exceeding 130 steps per minute over a predefined time interval or value designating a number of steps is lower than 20 steps per hour over a predefined time interval.

6. Method according to any one of 1-4 claims, where AF trigger occurrence instance data comprises threshold of a value designating a posture angle being calculated as

$$\theta = \tan^{-1} \frac{x_A x_R + y_A y_R + z_A z_R}{\sqrt{x_A^2 + y_A^2 + z_A^2}\sqrt{x_R^2 + y_R^2 + z_R^2}} ,$$

where $x_A$, $y_A$, and $z_A$ are the mean values of x-, y-, and z-axes for analysis interval, while $x_R$, $y_R$, and $z_R$ are the mean axis values for the reference interval, where first angle value threshold is <10°, second angle value threshold is >60°, third angle value threshold is between 10° and 60°.

7. Method according to any one of 1-4 claims, where AF trigger occurrence instance data comprises threshold of a value designating a sleep quality score including information on sleep latency, sleep duration, sleep stages, movement-caused restlessness and heart rate variability, respiratory rate, audible sound intensity levels, frequency ranges, energy peak frequencies, may be quantified in time-domain acoustic signals, e.g., with respect to increasing signal RMS, or by analysing the acoustic power spectrum.

8. Method according to any one of 1-4 claims, where AF trigger occurrence instance data comprises threshold of a value designating heart rate, heart rate variability, electrodermal activity, skin temperature, and PPG waveform morphology,
where one threshold value is designating sudden heart rate elevation by about 20% above the baseline resting heart rate and/or where second threshold value is heart rate variability reduced by about 20% below the baseline heart rate variability, and/or
occurrence instance data comprises threshold of a value designating acute emotional stress as a threshold value relating to resistance of skin to a small electrical current applied through two electrodes, where one threshold value relates to exceed 50% of baseline values, and/or
occurrence instance data comprises threshold of a value designating skin temperature, where the threshold value is a sudden drop by 0.1 or 0.2 Celsius, and/or

occurrence instance data comprises threshold of a value designating PPG waveform morphology such as the amplitude of forward pulse wave, the duration of diastole, and the time delay of the reflected pulse wave, the ratio of systolic and diastolic peak amplitudes.

9.  Method according to any one of 1-4 claims, where AF trigger occurrence instance data comprises threshold of a value designating bioimpedance spectroscopy-based body hydration.

10. Method according to any one of 1-4 claims, where AF trigger occurrence instance data comprises timestamp designating alcohol, caffeine, emotional stress, anxiety, large meals, cold beverages, high salt intake, cold foods, certain foods (e.g., onions, nuts, chocolate, ice cream, spicy food, garlic), bloating, constipation, emesis, relaxing, tiredness, infections.

11. Method according to any one of 1-4 claims, where AF occurrence instance data comprises threshold of a value designating heart rhythm irregularity, the absence of normal atrial activity representing P waves and the presence of abnormal atrial activity representing f-waves.

12. Method according to any one of previous claims, where characterizing (105) temporal AF episode occurrence patterns with respect to episode aggregation or/and clustering comprising expressing AF burden, a proportion of total monitoring time an individual being in AF during a monitoring period, by value from 0 to 1,
    where the duration of the longest AF episode or the number of AF episodes is taken during a predefined monitoring period of 1 day, or 3 days, or 1 week, or 2 weeks, or 1 month, or 3 months, or 6 months, or 1 year,
    where AF episode duration inequality index, such as the Gini coefficient, which approaches values close to 0 for the uniform distribution when AF episodes are of the similar duration, whereas approaches values close to 1 for widely different episode durations,
    where temporal AF episode concentration indexes, which take values closer to 1 for high temporal aggregation, inherent to patterns with a single short continuous AF episode or a single dense cluster of AF episodes, whereas values close to 0 indicate low temporal concentration, inherent to AF patterns with episodes evenly spread over the monitoring period of 1 day, or 3 days, or 1 week, or 2 weeks, or 1 month, or 3 months, or 6 months, or 1 year,
    where history-dependent point process modeling may be employed to characterize AF episode clustering using an alternating bivariate Hawkes model.

Fig. 1

FIG. 2

FIG. 3A

FIG. 3B

FIG. 4A

FIG. 4B

FIG. 4C

FIG. 4D

FIG. 4E

500

Continuous PPG monitoring

503

Alarm when AF is detected in PPG

505

Acquisition of multi-lead ECG

504

Collection of temporal AF occurrence pattern

502 502 502

AF episode AF episode AF episode

Continuous PPG

Multi-lead ECG

506 506 506

AF occurrence pattern

501

AF

non-AF

Monitoring period

**FIG. 5**

600

Temporal AF occurrence pattern characterization

AF

non-AF

Monitoring period

**Temporal information**

AF pattern

AF
non-AF

1 2 3 4 5 6 7
Monitoring time, days

601

AF burden

1

0
1 2 3 4 5 6 7
Day

602

Duration of longest episode

24

0
1 2 3 4 5 6 7
Day

603

Number of episodes

10

0
1 2 3 4 5 6 7
Day

605

**Episode distribution**

AF pattern

AF
non-AF

Actual distribution

Area₂

Area₁

Cumulative uniform distribution

Monitoring time

Line of equality

Lorenz curve

Area₁ Area₂

Monitoring time

Concentration index

$$\frac{Area_1}{Area_2}$$

Gini coefficient

$$\frac{Area_1}{Area_2}$$

604

**Episode clustering**

AF pattern

AF
non-AF

Alternating bivariate Hawkes model

Model parameters
$\mu_1$ $\mu_2$ $\beta_1$ $\beta_2$
$\alpha_{11}$ $\alpha_{12}$ $\alpha_{21}$ $\alpha_{22}$

Episode clustering
$\beta_1$ $\mu = \mu_1 / \mu_2$

606

**Fig. 6**

FIG. 7A

FIG. 7B

800

Load a temporal AF episode occurence pattern 801

Quantify the AF pattern 802

Load trigger data from a monitoring interval 803

Convert trigger data to a times-series signal 804

Compute the causality score between the suspected trigger and the AF pattern 805

The suspected trigger is not causal for AF 808

No ← Is a causal relationship identified (G > 0.9)? 806 → Yes

The suspected AF trigger is identified as causal for AF 807

Is there another suspected trigger? 809

Yes

No

End routine 810

FIG. 8

900

Characterization of
AF occurence patterns
105

Identified AF
triggers
108

Personal
recommendations
901

Interpretation of
changes in AF
occurence patterns
902

Fig. 9

1000

Existing risk factors
(e.g., obesity, heart
failure)
1001

Echocardiographic
parameters
1002

Identified AF
triggers
108

Data analysis and
identification of a
pathological chain
1004

Clinical consultation
and treatment
1005

Trigger-induced
alterations in
physiological systems
1003

Fig. 10

1100a

Obesity
1101

1103

1104

Sleep apnea
1105

Left ventricular
diastolic disfunction
1108

Secondary arterial
hypertension
1106

Left atrial dilatation
1109

Atrial premature
beats
1107

Left atrial scarring
1110

Atrial fibrillation
1102

Fig. 11A

1100b

Identified AF
triggers
108

⬇

Activated
sympathetic activity
1111

⬇

Increased blood
pressure
1112

⬇

Elevated pressure in
the left atrium
1113

⬇

Atrial premature
beats
1107

⬇

Atrial fibrillation
1102

Fig. 11B

1100c

Identified AF
triggers
108

Activated
parasympathetic
activity 1114

Decreased heart rate

1115

Escape beats

1116

Atrial fibrillation
1102

Fig. 11C

Fig. 12

Fig. 13

1400

Identified AF
triggers
108

Personal
recommendations
901

Interpretation of
changes in AF
occurence pattern
902

Risk factors
(e.g., obesity, heart
failure)
1402

Assessment of brain
stroke risk
1401

Clinical consultation
and treatment
1404

Module for collecting
temporal AF
occurence patterns
104

Module for
characterizing AF
occurence patterns
105

Morphology of left
atrial appendage
1403

Fig. 14

Europäisches
Patentamt

European
Patent Office

Office européen
des brevets

# EUROPEAN SEARCH REPORT

**Application Number**

EP 21 17 9681

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2016/204396 A1 (SAMSUNG ELECTRONICS CO LTD [KR]) 22 December 2016 (2016-12-22) * The whole document, in particular: Paragraphs [0015] - [0017], [0081], [0093], [0102], [0103], [0105], [0107] - [0108], [0112], [0116], [0122], [0124], [0125]. * | 1-12 | INV. G16H50/20 G16H50/30 |
| X,D | US 2020/229713 A1 (GOPALAKRISHNAN RAVI [US] ET AL) 23 July 2020 (2020-07-23) * The whole document, in particular: paragraphs [0010] - [0015], [0069] - [0076], [0101], [0112] - [0114]. * | 1-12 | |

**TECHNICAL FIELDS SEARCHED (IPC)**

G16H

The present search report has been drawn up for all claims

| Place of search | Date of completion of the search | Examiner |
|---|---|---|
| Munich | 30 November 2021 | Nagele, Stefan |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

 .....................................................................................

& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 21 17 9681

This annex lists the patent family members relating to the patent documents cited in the above-mentioned European search report.
The members are as contained in the European Patent Office EDP file on
The European Patent Office is in no way liable for these particulars which are merely given for the purpose of information.

30-11-2021

| Patent document cited in search report | | Publication date | Patent family member(s) | | Publication date |
|---|---|---|---|---|---|
| WO 2016204396 | A1 | 22-12-2016 | KR 20160147516 A<br>WO 2016204396 A1 | | 23-12-2016<br>22-12-2016 |
| US 2020229713 | A1 | 23-07-2020 | EP 3079571 A1<br>US 2015164349 A1<br>US 2015265164 A1<br>US 2017238814 A1<br>US 2019038149 A1<br>US 2020022594 A1<br>US 2020229713 A1<br>WO 2015089484 A1 | | 19-10-2016<br>18-06-2015<br>24-09-2015<br>24-08-2017<br>07-02-2019<br>23-01-2020<br>23-07-2020<br>18-06-2015 |

EPO FORM P0459

For more details about this annex : see Official Journal of the European Patent Office, No. 12/82

**EP 4 105 941 A1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 9420956 B2
- US 20200229713 A1
- US 20190008384 A1
- US 10726545 B2
- WO 2020104986 A2
- EP 3654347 A1

**Non-patent literature cited in the description**

- **GROH et al.** Patient-reported triggers of paroxysmal atrial fibrillation. *Heart Rhythm,* July 2019, vol. 16.7, 996-1002
- **HANSSON et al.** Arrhythmia-provoking factors and symptoms at the onset of paroxysmal atrial fibrillation. *BMC Cardiovascular Disorders,* December 2004, vol. 4.1, 13
- **SOLOŠENKO et al.** Detection of atrial fibrillation using a wrist-worn device. *Physiological Measurement,* February 2019, vol. 40.2, 025003
- *European Heart Journal,* February 2021, vol. 42.5, 373-498
- **CHARITOS et al.** Atrial fibrillation density: A novel measure of atrial fibrillation temporal aggregation for the characterization of atrial fibrillation recurrence pattern. *Applied Cardiopulmonary Pathophysiology,* January 2013, vol. 17, 3-10
- **HENRIKSSON et al.** Modeling and estimation of temporal episode patterns in paroxysmal atrial fibrillation. *IEEE Transactions on Biomedical Engineering,* January 2021, vol. 68.1, 319-329
- **VOSKOBOINIK et al.** Alcohol abstinence in drinkers with atrial fibrillation. *New England Journal of Medicine,* January 2020, vol. 382.1, 20-28
- **CHUNG et al.** Lifestyle and risk factor modification for reduction of Atrial Fibrillation. *Circulation,* April 2020, vol. 141.16, e750-e772